# EUROPEAN PATENT APPLICATION

(11) **EP 3 086 277 A1**
(43) Date of publication of application: **26.10.2016**
(21) Application number: 14871515.4
(22) Date of filing: 15.12.2014
(51) Int. Cl.: G06Q 50/22

(54) **CLINICAL PATH MANAGEMENT SERVER**

(30) Priority: 19.12.2013 JP 2013262556
(71) Applicant: Fujifilm Corporation, Minato-ku Tokyo 106-8620 (JP)
(72) Inventor: OHTA, Yasunori, Ashigarakami-gun Kanagawa 258-8538 (JP); UEDA, Satoshi, Tokyo 106-8620 (JP); KUDOU, Yuuya, Tokyo 106-8620 (JP); MATSUMASA, Hironori, Tokyo 106-8620 (JP)
(74) Representative: Klunker . Schmitt-Nilson . Hirsch
(86) International application number: PCT/JP2014/083117
(87) International publication number: WO 2015/093431

(57) **Abstract**

The clinical path management server has a clinical path database (CPDB) (32), an access request reception unit (41), a determination unit (42), and a warning unit (43). The CPDB (32) stores a clinical path, in which schedules and work results relating to a plurality of medical practices are recorded in time series, such that the clinical path can be searched. The access request reception unit (41) receives an access request with respect to the clinical path, from the operator terminal (14). The determination unit (42) determines the presence or absence of omission of input of work results of the past medical practices of which the input should be already completed at a point in time when the access request is received, when the access request is received. The warning unit (43) issues a warning in a case where it is determined that there is omission of input.

## Description

### CROSS-REFERENCE TO RELATED APPLICATIONS

This application is a Continuation of PCT International Application No. PCT/JP2014/083117 filed on December 15, 2014, which claims priority under 35 U.S.C §119(a) to Japanese Patent Application No. 2013-262556 filed December 19, 2013. The above application is hereby expressly incorporated by reference, in its entirety, into the present application.

### BACKGROUND OF THE INVENTION

### 1. Field of the Invention

The present invention relates to a clinical path management server which manages a clinical path in which medical treatment information of a patient is recorded.

### 2. Description Related to the Prior Art

A clinical path, in which schedules and work results of a plurality of medical practices performed on a patient are recorded in time series, is known. The clinical path is created for each patient and is used in order to check medical treatment history of a patient or to determine a medical treatment policy (for example, refer to JP2003-108661A). In JP2003-108661A, a clinical path management system which manages a clinical path is disclosed. The system of JP2003-108661A has input reception means for receiving an input of a work result relating to a nursing treatment practice which is one of the medical practices; and storage means which stores a clinical path to which the work result is input. When one medical practice is finished, a nurse who is a person in charge of work of a nursing treatment practice inputs the work result thereof into the system through an input terminal. In this system, a function of detecting whether or not all necessary matters to be stated regarding the work result are input while inputting the work result relating to the one medical practice, and issuing a warning using a voice, a highlighting on an input screen, or the like in a case where the necessary matters are not input is provided. It is possible to prevent omission of input of necessary matters to be stated using this function.

In addition, an output device of clinical trial data which collects clinical trial data of a medicine and outputs the collected clinical trial data by performing administration of a medicine to a patient which is one medical practice is known (for example, refer to JP2004-348271A). For example, administration of a medicine is periodically performed, once a day or once a week. The clinical trial data are periodically collected as work results obtained by performing administration of a medicine, and are input to the output device by a person in charge of work each time. In this output device, a detection unit which detects whether or not the clinical trial data are periodically recorded is provided. The detection unit periodically performs a detection operation, for example, once a week, and detects whether or not the clinical trial data are periodically input thereto. In a case where omission of input of clinical trial data is detected, warning of the information is given by a message or the like.

A clinical path is importantly used to check medical treatment history of a patient or to determine a medical treatment policy. Therefore, it is preferable to prevent omission of input of work result. In order to prevent the omission of input of a work result, it is preferable to immediately perform input of a work result when a person in charge of work performs one medical practice. However, in very busy medical sites, the input of a work result tends to be delayed even if the medical practice is performed. If the input of a work result is delayed, there is a concern that omission of input of a work result is left due to forgetfulness of a person in charge of work. In some cases, a work result of one medical practice is referred to when performing a medical practice to be performed thereafter. Therefore, it is not preferable that omission of input is left for a long period of time.

In JP2003-108661A, it is possible to perform detection or warning of omission of input of necessary matters to be stated, while inputting a work result relating to one medical practice. However, in a case where the input of the work result is delayed once, the detection and the warning of omission of input are not performed thereafter. Therefore, there is a concern that the omission of input of the work result which has been delayed may be left for a long period of time.

In contrast, if an operation of detecting omission of input is periodically performed as in JP2004-348271A, it is possible to perform detection and warning of omission of input of work results of past medical practices. However, in a case where the operation of detecting omission of input is periodically performed, there is a concern that the a warning may be issued at an inappropriate timing when a person in charge of work cannot immediately perform input of a work result, for example, at a timing when the person in charge of work performs a medical practice. If the timing of a warning is inappropriate, input of a work result is delayed again, and therefore, there is a problem in that the warning is not effective. In addition, if the warning is issued at an inappropriate timing, in some cases, the medical practice which is being performed needs to be interrupted. If the medical practice is interrupted, there is a concern that the working efficiency of the medical practice which is being performed is impaired or an operation error may be caused.

### SUMMARY OF THE INVENTION

An object of the present invention is to provide a clinical path management server which prevents omission of input of work results to a clinical path from being left for a long period of time.

In order to achieve the above-described object, the clinical path management server of the present invention comprises a clinical path database, an access request reception unit, a determination unit, and a warning unit. The clinical path database stores a clinical path, in which schedules and work results relating to a plurality of medical practices performed on a patient are recorded in time series, such that the clinical path is searchable. The access request reception unit receives an access request with respect to the clinical path from a terminal. The determination unit determines the presence or absence of omission of input of work results of the past medical practices of which the input should have been already completed at a point in time when the access request with respect to the clinical path is received, upon receiving the access request. The warning unit warns that there is omission of the input in a case where it is determined that there is omission of the input.

It is preferable that the access request includes at least one of a browsing request of the clinical path, an editing request for inputting the work results to the clinical path, and an end request for finishing the browsing or the edition. In addition, it is preferable that the access request is the editing request, and the past medical practices are medical practices performed before the medical practice corresponding to the editing request. In addition, it is preferable that, in the clinical path which is a target of the access request, the determination unit extracts the medical practice, of which the scheduled date and time of implementation is prior to the date and time when the access request is received, as the past medical practice, and determines the presence or absence of the omission of input by checking an item of an input condition of the extracted past medical practice.

It is preferable that the warning unit issues a warning to the terminal of a request source of the access request. In addition, it is preferable that the warning unit issues the warning by transmitting a warning screen on which a medical practice, in which there is omission of the input, is displayed. In addition, it is preferable that a name of a person in charge of work of the medical practice is displayed on the warning screen. In addition, it is preferable that the clinical path management server further includes an address storage unit which stores in advance an address of a person in charge of work of the medical practice recorded in the clinical path, and it is preferable that the warning unit issues a warning to the person in charge of work of the medical practice, in which there is omission of the input, with reference to the address storage unit, in addition to the terminal of the request source.

The present invention determines the presence or absence of omission of input of work results of past medical practices when an access request to a clinical path is received, and issues a warning at that timing. The warning is issued when the access request to the clinical path is received. Therefore, the possibility that input of a work result is delayed again is low compared to a timing at which other works, for example, in the middle of performing a medical practice are performed, thereby enhancing effectiveness. For this reason, the omission of input is prevented from being left for a long period of time.

### BRIEF DESCRIPTION OF DRAWINGS

For more complete understanding of the present invention, and the advantage thereof, reference is now made to the subsequent descriptions taken in conj unction with the accompanying drawings, in which:
Fig. 1 is a schematic view of a clinical path management system of a first embodiment of the present invention;
Fig. 2 is a configuration view of a clinical path management server;
Fig. 3 is an explanatory view of a clinical path database;
Fig. 4 is an explanatory view of a clinical path display screen;
Fig. 5 is an explanatory view of records of a medical practice;
Fig. 6 is a functional schematic view of the clinical path management server;
Fig. 7 is an explanatory view relating to a function of extracting past medical practices;
Fig. 8 is an explanatory view of the difference in records due to the presence or absence of omission in entry;
Fig. 9 is an explanatory view of a warning screen;
Fig. 10 is a flowchart of the entirety of the first embodiment;
Fig. 11 is an explanatory view of an aspect of information input in a second embodiment;
Fig. 12 is an explanatory view of address information in a third embodiment;
Fig. 13 is a schematic view relating to a warning of the third embodiment; and
Fig. 14 is an explanatory view relating to an extraction standard of past medical practices.

### DESCRIPTION OF THE PREFERRED EMBODIMENTS

### [First Embodiment]

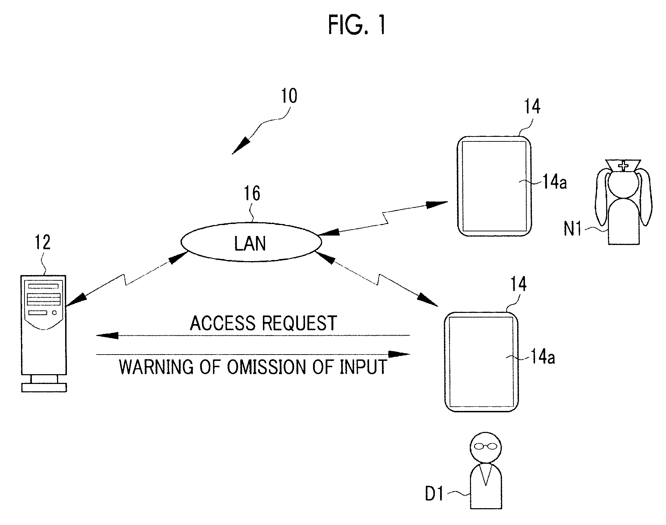

As shown in Fig. 1, a clinical path management system (hereinafter, referred to as a clinical path (CP) management system) 10 has a clinical path management server (hereinafter, referred to as a CP management server) 12 and an operator terminal 14. All of the CP management server 12 and the operator terminal 14 are provided in an X hospital, and are connected to each other through a local area network (LAN) 16, which is an in-hospital network which is constructed in the X hospital, so as to communicate information.

The operator terminal 14 is a terminal which an operator such as a doctor D1 or a nurse N1, who performs a medical practice on a patient, uses. The operator terminal 14 is, for example, a tablet type portable terminal having a wireless communication function, and is provided with a touch panel type display 14a which functions as a display unit or an input unit of information. In the operator terminal 14, an operating system or software such as a WEB browser is installed, and the software is executed by a central processing unit (CPU) or a memory. The operator terminal 14 can access to the CP management server 12 via the LAN 16 through the WEB browser.

In a case where the operator terminal 14 is connected to the LAN 16, the operator terminal 14 is actually connected to the LAN 16 through an access point as a radio repeater. The display 14a displays an operation screen, receives various operation instructions or an input of information, and displays information, such as various screens, distributed from the CP management server 12. If the doctor D1 or the nurse N1 carries the operator terminal 14, it is possible to browse or edit a clinical path of a patient under charge at any place accessible to the LAN 16. As the information distributed from the CP management server 12, a CP display screen, a warning screen, or the like on which CP is displayed is included as will be described below.

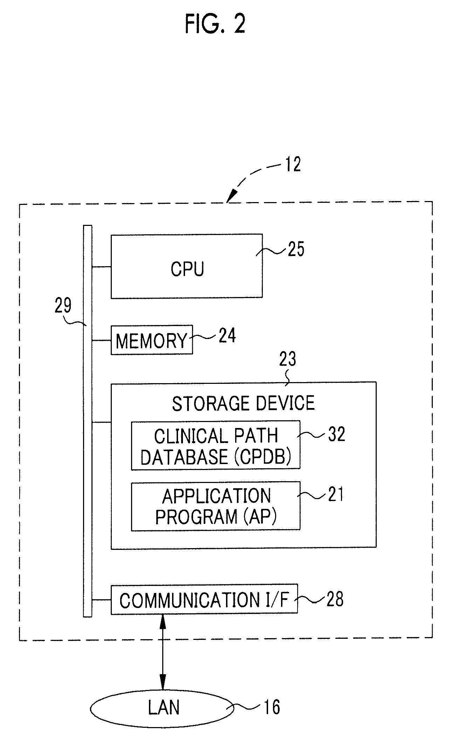

As shown in Fig. 2, the CP management server 12 is constituted by installing an application program (AP) 21 for operating the CP management system 10, based on a personal computer or a workstation. In the CP management server 12, a storage device 23, a memory 24, a CPU 25, and a communication I/F 28 which are connected to each other through a data bus 29.

The storage device 23 is a device in which various data are stored, and is constituted of, for example, a hard disk drive. In the storage device 23, a clinical path database (hereinafter, referred to as a CPDB) 32 is stored in addition to a control program or the AP 21 such as software for a CP system.

The memory 24 is a work memory which is used for the CPU 25 to execute processing. The CPU 25 integrally controls each unit of a computer by loading the AP 21 which has been stored in the storage device 23 in the memory 24 and executing processing according to a program.

The communication I/F 28 is a communication unit for connecting the CP management server to the LAN 16. The communication I/F 28 communicates with the operator terminal 14 and each of other terminals provided in the X hospital, through the LAN 16. The CP management server 12 receives various requests such as information distribution requests from the operator terminal 14 and each of the other terminals through the communication I/F 28, and executes processing according to the content of the requests after receiving the received requests. In addition, the CP management server 12 issues a command to the operator terminal 14 and each of the other terminals through the communication I/F 28.

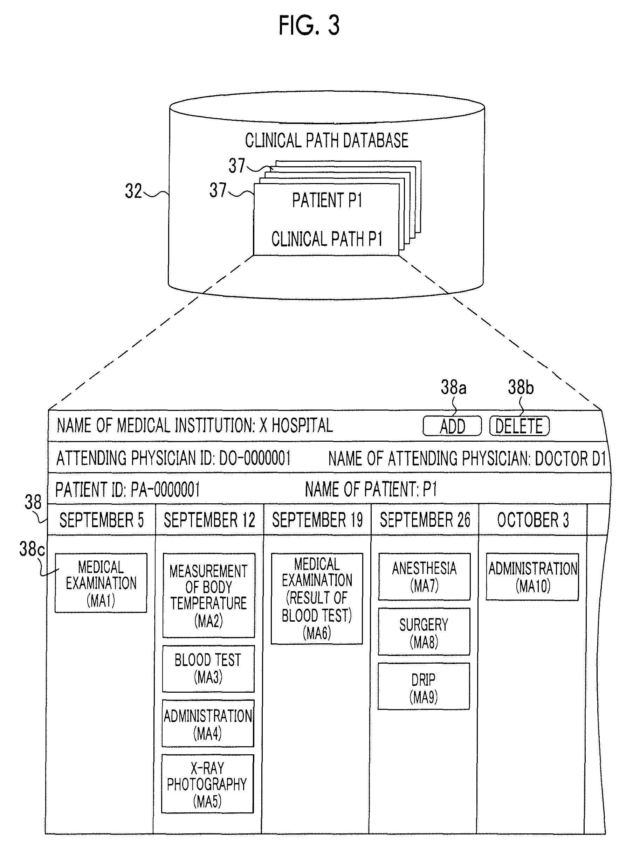

The CPDB 32 is a database in which a plurality of pieces of clinical path data (hereinafter, referred to as CP data) 37 created for each patient are stored as shown in Fig. 3. The CP data 37 is displayed on a display 14a of the operator terminal 14 after being distributed as screen data in which each display item is laid out as shown in, for example, a CP display screen 38.

The CP data 37 is data of medical treatment schedule in which schedules of medical treatment (diagnosis and treatment) performed on a patient and medical treatment information relating to results are recorded in time series in the X hospital. In the medical treatment information, schedules of medical practices such as a medical examination, inspection, surgery, and administration, and work results (results), such as inspection results, medical examination results, and administration results, of the medical practices are included. In addition, the clinical path is information created for each patient. Therefore, patient information such as patient ID, the name of a patient, age, and sex, the name or ID of an attending physician who is in charge of the patient, and the like are recorded in the CP data 37.

The CP management server 12 distributes the CP data 37 to the operator terminal 14 in accordance with a browsing request from the operator terminal 14. When the CP data 37 is received by the operator terminal 14, the CP display screen 38 is displayed on the display 14a. Therefore, an operator such as the doctor D1 and the nurse N1 can browse the clinical path. In addition, in a case where a clinical path is newly created by the operator in the operator terminal 14 or edition, such as input, change, or deletion, of data is performed on the created clinical path, the CP management server 12 updates the CPDB 32 by receiving a command of new creation or edition from the operator terminal 14.

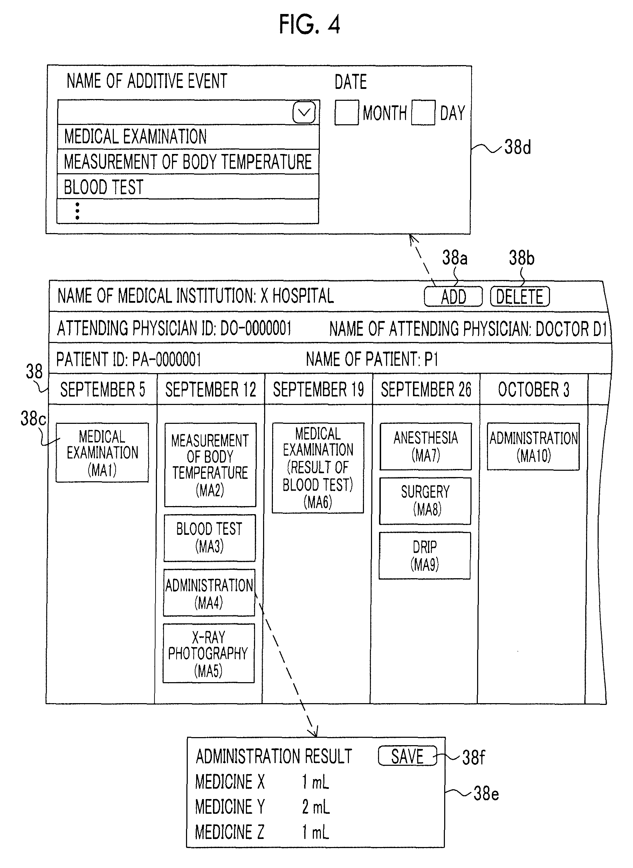

As shown in Fig. 4, in the CP display screen 38, medical practices such as a medical examination, inspection, surgery, and administration are displayed for each date on which the medical practices are performed. For example, display columns of medical practices, in which the dates such as September 5, September 12, September 19, September 26, and October 3 are given, are provided. In addition, medical practices such as a medical examination (MA1), body temperature measurement (MA2), a blood test (MA3), administration (MA4), X-ray photography (MA5), a medical examination (report of a blood test) (MA6), anesthesia (MA7), surgery (MA8), a drip (MA9), and administration (MA10) are displayed in the display columns corresponding to the respective dates on which the medical practices are performed. Here, medical act (MA) is a code which is given for convenience in order to simply indicate each of the medical practices. In the CP display screen 38, an add button 38a used for adding a scheduled medical practice to the CP data 37 and a deletion button 38b used for deleting a canceled medical practice are provided. In addition, the display units of each of the medical practices MA1 to MA10 are buttons 38c, and when each of the buttons 38c is operated, it is possible to display or edit the detailed contents of the medical practices.

When the add button 38a is selected in the CP display screen 38, an item add screen 38d is pop-up displayed. Spaces, in which a name of a medical practice and a date to which the medical practice is performed are input after being selected, are provided in the item add screen 38d. By selecting and inputting them, the schedule of the selected medical practice is added to a display column of a designated date. In contrast, when the deletion button 38b is selected, the system enters a medical practice deletion mode, and the medical practice is deleted from the display column by selecting a button 38c corresponding to the medical practice to be deleted under the medical practice deletion mode.

When the button 38c is selected, a detailed display screen 38e which displays the details of the corresponding medical practice is pop-up displayed. In the detailed display screen 38e, display or edition of the detailed contents of the medical practice is performed. In addition, there is an edition save button 38f in an upper right portion of the detailed display screen 38e. When the edition save button 38f is selected, an input request to be described below is made with respect to the CP management server 12. If this input request is permitted, the CP data 37 in the CPDB 32 is edited, and the updated contents of the CP data are saved.

In the edition contents relating to the medical practice, a result of work after being performed, which is information of a result of the medical practice is included. For example, when the button 38c of administration (MA4) on September 12 is selected, the detailed display screen 38e is displayed. In the detailed display screen 38e, information that 1 mL of a medicine X, 2 mL of a medicine Y, and 1 mL of a medicine Z are administered is input as a work result of the administration (MA4). In a case where image data or the like is input as a work result, address information or the like of a storage destination of the image data is input.

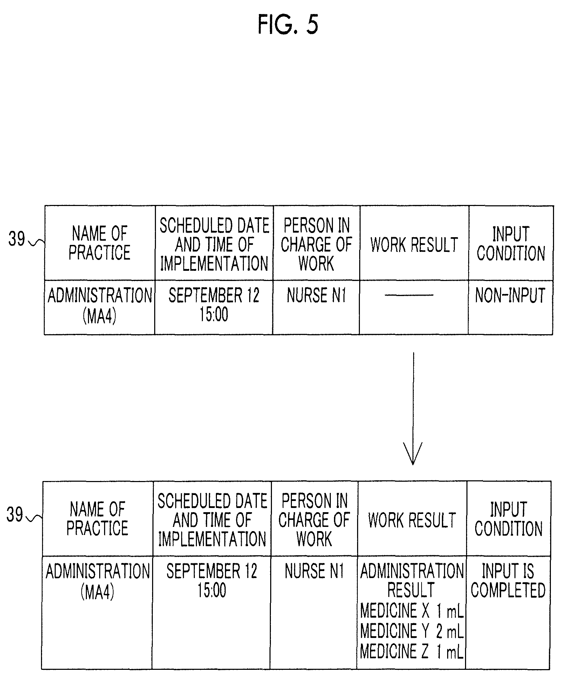

As shown in Fig. 5, in the CP data 37, information pieces relating to each of the medical practices are recorded as records 39 for each case. In the records 39, there are items such as the name of a medical practice (the name of a practice), the scheduled date and time (scheduled date and time of implementation) on which the medical practice is performed, a person in charge of work who performs the medical practice, a work result of the medical practice, and an input condition. The records 39 shown in Fig. 5 are examples of the administration (MA4). The item "input condition" represents an input condition of a work result. In a case where no work result is input as shown in the upper records 39, the input condition is recorded as "non-input". In a case where a work result is input as shown in the lower records 39, the input condition is recorded as "input is completed".

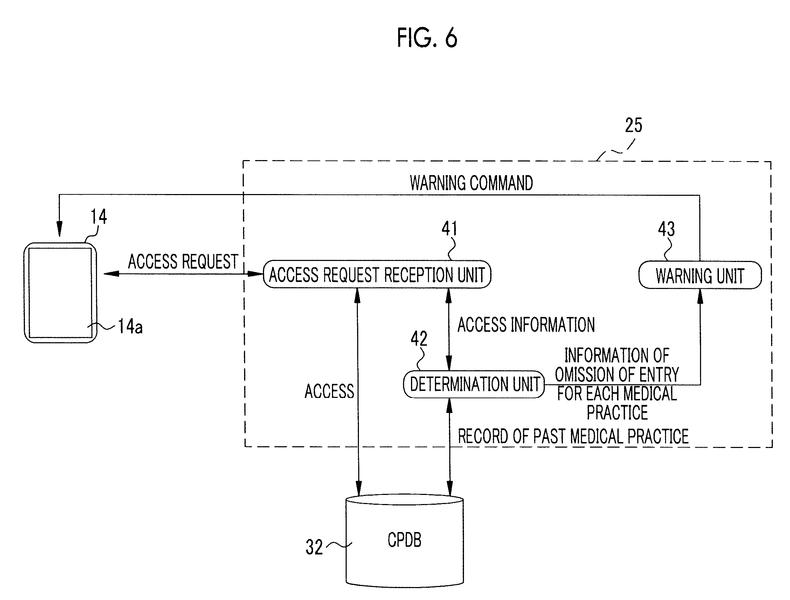

When the AP 21 is executed, the CPU 25 of the CP management server 12 functions as an access request reception unit 41, a determination unit 42, and a warning unit 43 as shown in Fig. 6.

The access request reception unit 41 is a functional unit which executes processing according to an access request received from the operator terminal 14. In the access request to the CPDB 32, there is a browsing request in which distribution of the CP data 37 is requested in order to browse the CP data 37, and an editing request in which the CP data 37 is edited in the operator terminal 14 and update of the CPDB 32 in the CP data 37 which has been edited.

Here, information which specifies the CP data 37 of an access object such as a patient ID is included in the access request, and authentication information for authenticating whether an access request source has an access right is attached to the access request. Therefore, the access request and the attached authentication information are input to the access request reception unit 41. The authentication information is an operator ID (a doctor ID or a nurse ID) which is allocated in advance to each operator such as a doctor D1 or a nurse N1, or a password. In addition, the CPU 25 has a system timer and records the dates and times of access requests as an access history.

The access request reception unit 41 permits an access in a case where the access request source has an access right according to the request contents. The access request reception unit refuses a request in a case where the access request source does not have an access right. For example, in a case where a browsing request is permitted, the access request reception unit 41 distributes the CP data 37 after reading the CP data 37 from the CPDB 32. In a case where an editing request is permitted, the access request reception unit 41 updates the CP data 37 in the CPDB 32 with CP data 37 which has been uploaded from the operator terminal 14. In addition, when an access is permitted, the access request reception unit 41 transmits access information, which indicates that there has been an access with respect to the CP data 37, to the determination unit 42. Specifically, a data ID of the CP data 37 of an access object specified by a patient ID or the like, the date and time of an access, and the like are included in the access information.

The determination unit 42 is a functional unit which determines the presence or absence of omission of input of a work result with respect to each past medical practice for which input of a work result to the CP data 37 is already completed at a point in time when an access request is received. When access information is received, the determination unit 42 extracts records 39 relating to past medical practices of which the scheduled date and time of implementation is prior to the date and time of the access in the CP data 37 of an access obj ect. Next, the determination unit 42 determines the presence or absence of omission of input of a work result by checking the item of the input condition of the extracted records 39.

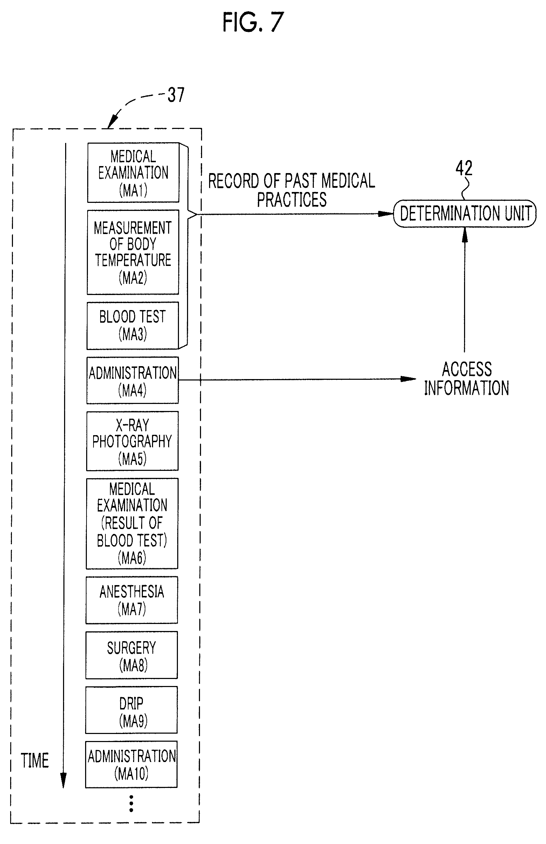

For example, as shown in Fig. 7, in a case where the date and time of an access is the scheduled date and time of implementation of the administration (MA4), records 39 of past three medical practices (MA1 to MA3) which are performed prior to the scheduled date and time of implementation of the administration (MA4) are extracted. A work result needs to be input at a point in time when a medical practice is finished. Therefore, in a case where no work result is input with respect to a medical practice after the scheduled date and time of implementation, it is considered that there is omission of input of the work result with respect to the medical practice.

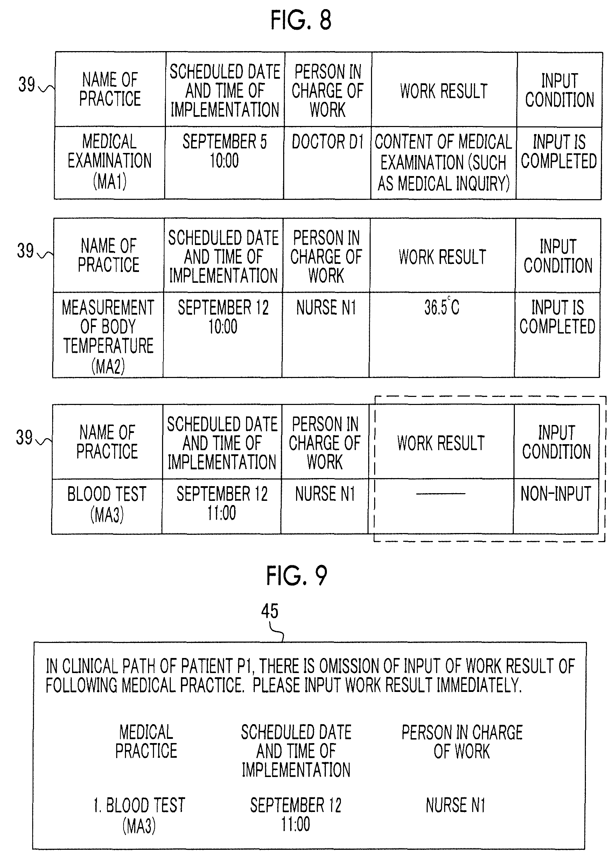

As shown in Fig. 8, work results are input to the records 39 of medical examination (MA1) and measurement of body temperature (MA2) out of the records 39 of extracted three cases. However, no work result is input to the records 39 of a blood test (MA3) as shown by a dotted line. The determination unit 42 determines that there is omission of input of the work result of the blood test (MA3) by checking the input condition of each of the records 39.

In a case where the determination unit 42 determines that there is omission of input of a work result, the determination unit issues a warning command so as to make the warning unit 43 issue a warning. When the warning unit 43 receives the warning command, the warning unit issues a warning by, for example, transmitting a warning screen 45 shown in Fig. 9 to the operator terminal 14 as an access request source. On the warning screen 45, the name of a medical practice in which there is omission of input, the scheduled date and time of implementation, and the name of a person in charge of work are displayed. In this example, information of omission of input of one case is displayed. However, in a case where there are information pieces of omission of input of a plurality of cases, the information pieces of the plurality of cases are displayed.

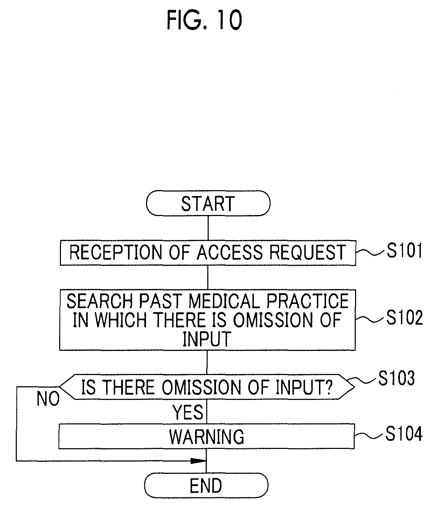

Hereinafter, the action caused by the above-described configuration will be described while referring to a flowchart shown in Fig. 10. For example, in a case where the nurse N1 performs administration (MA4) on a patient P1, the nurse N1 accesses to a clinical path of the patient P1 by connecting to the CP management server 12 using the operator terminal 14 in order to perform a work result thereof. For example, in a case of inputting the work result of the administration (MA4), the nurse N1 inputs an instruction of an editing request with respect to the CP data 37 of the patient P1 from an operation screen of the operator terminal 14. The operator terminal 14 transmits the editing request to the CP management server 12 based on the input instruction. In the CP management server 12, the access request reception unit 41 receives the editing request (S101).

When the access request reception unit 41 receives the editing request, whether or not the access request source has an access right is determined based on authentication information. As a result of the determination, in a case where the access request source has an edition right, the edition right is permitted and the CP data 37 of the designated patient P1 is distributed to the operator terminal 14. In the operator terminal 14, the CP data 37 is displayed as the CP display screen 38. The nurse N1 inputs the work result of administration (MA4) by operating the CP display screen 38. When the edition is finished, the edition contents are uploaded to the CP management server 12 from the operator terminal 14, and the CP data 37 in the CPDB 32 is updated. Furthermore, in a case where there is an editing request, the access request reception unit 41 transmits access information to the determination unit 42.

When the determination unit 42 receives the access information, the determination unit acquires the CP data 37 of the patient P1 of an access object from the CPDB 32. The determination unit 42 extracts records 39 of past medical practices (MA1 to MA3), of which the scheduled date and time of implementation is prior to the date and time of the access in the clinical path of the patient P1, and searches a medical practice in which there is omission of input of a work result by checking the input condition of each of the records 39 (S102).

In a case where there is a past medical practice in which there is omission of input (YES in S103), the determination unit 42 issues a warning through the warning unit 43 (S104). When the warning unit 43 receives a warning command from the determination unit 42, the warning unit transmits the warning screen 45 on which the name of a medical practice, in which there is omission of input, the scheduled date and time of implementation, and the person in charge of work are displayed as shown in Fig. 9, is transmitted to the operator terminal 14 of the access request source. In a case where there is no past medical practice in which there is omission of input (NO in S103), the determination unit 42 finishes the determination processing without issuing a warning.

In this manner, in the present invention, a warning is issued in a case where it is determined that there is omission of input after determining the presence or absence of the omission of input of a work result of a past medical practice of which the input should be already completed, by checking the input condition of a clinical path at a point in time when an access request to the clinical path is received. The timing at which the clinical path is accessed can be called a timing at which it is easy to perform edition of the clinical path compared to a timing at which other works, for example, in the middle of performing a medical practice, are performed. If a warning is issued at that timing, the possibility that input of a work result is delayed again is low. Therefore, it is possible to highly effectively issue a warning. For this reason, the omission of input of a work result is prevented from being left for a long period of time.

In addition, a person in charge of work who accesses to a clinical path is a doctor or a nurse who is in charge of a patient of the clinical path. Therefore, there is a high possibility that the person in charge of work who accesses to the clinical path may be a person in charge of work of a medical practice in which there is omission of input. For this reason, if a warning is issued to the operator terminal 14 of an access request source, there is a high possibility that the warning is directly issued to the person in charge of work who needs to input the work result. Even if a person in charge of work who has received a warning is a different person from the person in charge of work of the medical practice in which there is omission of input, there is a high possibility that both people in charge of work may be members of a team in charge of the same patients. Therefore, there is a high possibility that the contents of the warning may be transmitted to the person in charge of work of the medical practice in which there is omission of input.

### [Second Embodiment]

In the above-described embodiment, the example of the editing request with respect to a clinical path has been shown as an example of an access request, but a browsing request with respect to a clinical path may be shown as the example of the access request. In addition, the edition with respect to a clinical path is not limited to a mode of manual input, and a mode of automatic input from various medical treatment devices may be used.

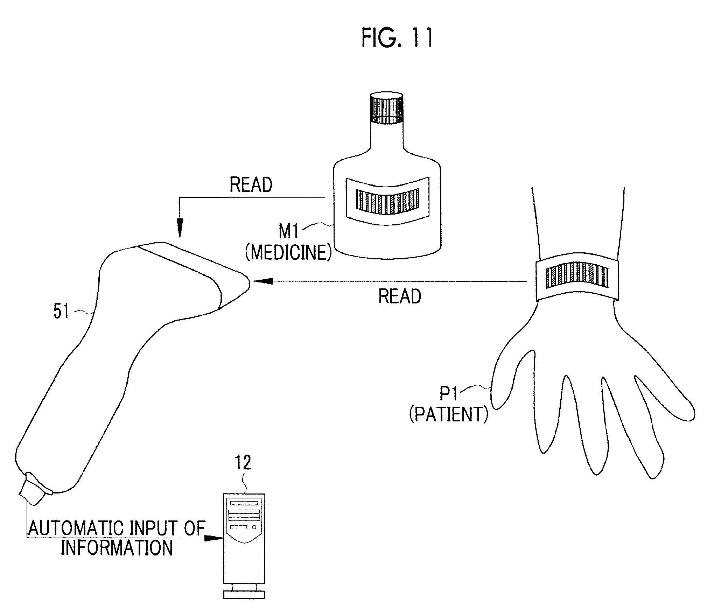

As the medical treatment device, there is, for example, a bar code reader 51 which reads a bar code for patient identification or a bar code for medicine identification as shown in Fig. 11. A mode in which the information of the patient P1 is read using the bar code reader 51 and the information, which has been automatically read from the bar code reader 51, is input to a clinical path may be used.

In Fig. 11, the bar code reader 51 reads, for example, a bar code on a band mounted on the wrist of the patient P1 and a bar code on a seal attached to a medicine M1 which is scheduled to be administered to the patient P1. The bar code reader 51 and the operator terminal 14 are communicably connected to each other, and the data read by the bar code reader 51 are transmitted to the CP management server 12 via the operator terminal 14. Browsing or edition of the clinical path of the patient P1 is performed in the operator terminal 14, and therefore, the information of the patient P1 which has been read by the bar code reader 51 is automatically input to the clinical path of the corresponding patient P1. The CP management server 12 receives the information read by the bar code reader 51 from the operator terminal 14. The access request reception unit 41 receives an access request and the determination unit 42 executes determination processing relating to the above-described omission of input.

An example of the editing request or the browsing request has been described as the access request. However, the access request may include an end request which ends browsing or edition in addition to or instead of the editing request or the browsing request.

### [Third Embodiment]

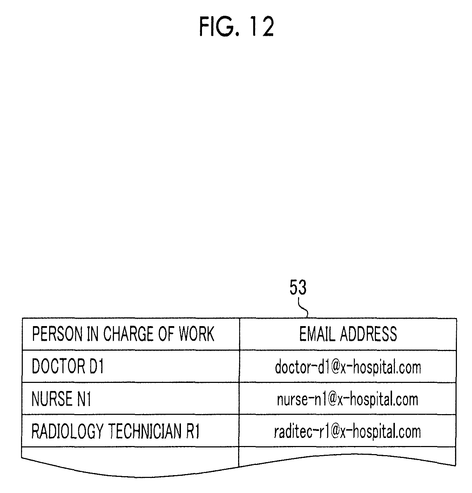

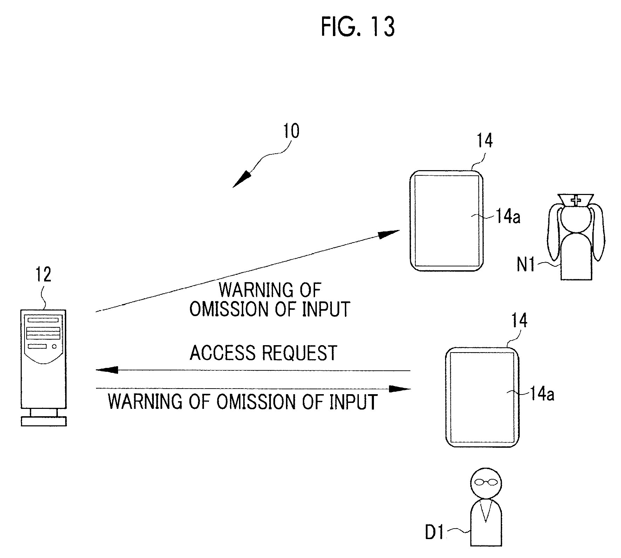

In addition, the example in which a warning destination is set to the operator terminal 14 of the access request source has been described. However, a warning may be transmitted to an address of a person in charge of work of a medical practice in which there is omission of input of a work result, in addition to or instead of the access request source. As shown in Figs. 5 and 8, a person in charge of work of each medical practice is input to the clinical path. As shown in Fig. 12, address information 53 in which an email address of each person in charge of work is previously set is stored in the storage device 23 of the CP management server 12. The storage device 23 functions as an address storage unit. The warning unit 43 reads an email address from the address information 53 , corresponding to a person in charge of work of a medical practice in which there is omission of input, with reference to the address information 53 and transmits the warning screen 45 to the read email address. For example, in a case where there is omission of input in a medical practice that the doctor D1 is in charge of and in a medical practice that the nurse N1 is in charge of, a warning screen is transmitted to both doctor D1 and nurse N1 as shown in Fig. 13. By doing this, it is possible to reliably issue a warning to the person in charge of work of the omission of input.

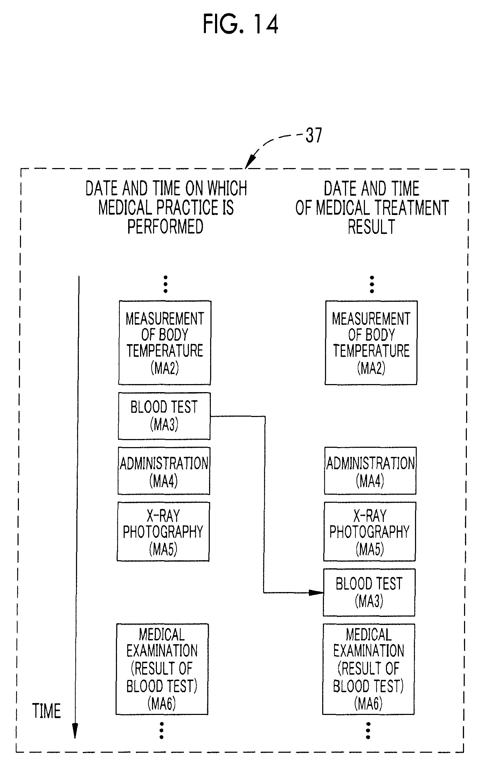

In the above-described embodiments, past medical practices, of which the input should be already completed at a point in time when an access request is received, are searched at a point in time when an access request is received on the basis of the date and time on which the medical practices are performed, but the present invention is not limited thereto. In addition, it is possible to set, for example, the date and time on which a result of medical treatment comes out as a standard. For example, in some cases, a special machine or a special medicine is required for a blood test depending on the item to be tested. Therefore, in the cases, the test is often outsourced to a test institution by sending collected blood to the test institution after only collecting blood in a hospital. In this case, it takes time until a test result is input as a work result by obtaining the test result after the start of the blood test. For this reason, as shown in Fig. 14, it is preferable to set the system such that a medical practice is searched as a past medical practice if the date and time on which a result of medical treatment comes out is passed rather than to set the system such that a medical practice is searched as a past medical practice if the date and time on which the medical practice is performed.

In the present embodiments, the tablet type portable terminal having a wireless communication function has been used as the operator terminal 14, but the present invention is not limited thereto. For example, a desktop type computer or a notebook type computer which has a communication function may be used.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. A clinical path management (12) server comprising:
a clinical path database (32) that stores a clinical path, in which schedules and work results relating to a plurality of medical practices performed on a patient are recorded in time series, such that the clinical path is searchable;
an access request reception unit (41) which receives an access request with respect to the clinical path from a terminal (14);
a determination unit (42) which determines the presence or absence of omission of input of work results of the past medical practices of which the input should have been already completed at a point in time when the access request with respect to the clinical path is received, upon receiving the access request; and
a warning unit (43) which warns that there is omission of the input in a case where it is determined that there is omission of the input.

2. The clinical path management server according to claim 1, wherein the access request includes at least one of a browsing request of the clinical path, an editing request for inputting the work results to the clinical path, and an end request for finishing the browsing or the edition.

3. The clinical path management server according to claim 2, wherein
the access request is the editing request, and
the past medical practices are medical practices performed before the medical practice corresponding to the editing request.

4. The clinical path management server according to claim 1, wherein the determination unit extracts the medical practice, of which the scheduled date and time of implementation is prior to the date and time when the access request is received, in the clinical path which is a target of the access request, as the past medical practice, and the determination unit determines the presence or absence of the omission of input by checking an item of an input condition of the extracted past medical practice.

5. The clinical path management server according to claim 1, wherein the warning unit issues a warning to the terminal of a request source of the access request.

6. The clinical path management server according to claim 5, wherein the warning unit issues the warning by transmitting a warning screen on which a medical practice, in which there is omission of the input, is displayed.

7. The clinical path management server according to claim 6, wherein a name of a person in charge of work of the medical practice is displayed on the warning screen.

8. The clinical path management server according to claim 5, further comprising an address storage unit (23) which stores in advance an address of a person in charge of work of the medical practice recorded in the clinical path, wherein
the warning unit issues a warning to the person in charge of work of the medical practice, in which there is omission of the input, with reference to the address storage unit, in addition to the terminal of the request source.
